# EUROPEAN PATENT SPECIFICATION

(11) **EP 0 506 030 B1**
(45) Date of publication and mention of the grant of the patent: **26.07.1995**
(21) Application number: 92105160.3
(22) Date of filing: 25.03.1992
(51) Int. Cl.: A61B 8/06

(54) **Hydrodynamic system for blood flow measurement**
Hydrodynamische Anordnung zur Messung der Blutströmung
Système hydrodynamique destiné à la mesure de l'écoulement sanguin

(30) Priority: 25.03.1991 YU 532/91
(43) Date of publication of application: 30.09.1992
(73) Proprietor: Breyer, Branco, Dr., YU-41000 Zagreb, Croatia (YU); Ferek-Petric, Bozidar, YU-41000 Zagreb, Croatia (YU)
(72) Inventor: Breyer, Branco, Dr., YU-41000 Zagreb, Croatia (YU); Ferek-Petric, Bozidar, YU-41000 Zagreb, Croatia (YU)
(74) Representative: Blumbach, Kramer & Partner

(56) References cited:
- EP-A- 0 474 957
- US-A- 4 237 729
- US-A- 4 757 821

## Description

The present invention is related to medical technology and to blood flow measurement with special attention to embodiments suitable for cardiac pacemaker operation control.

Measurement of blood flow velocity in medicine can be transcutaneous (through the skin) or intraluminal (directly within the flow). The present invention relates to the intraluminal flow measurement.

Intraluminal flow measurement is needed in all invasive cardiovascular procedures e.g. catheterisation, pacemaker application and cardiovascular surgery.

At the present such measurements are done on the basis of Doppler methods as well as thermodilution methods. Measurements using the Doppler effect function by means of transmission of ultrasound (pulse or continuous wave) into the blood stream and by means of detection of the Doppler frequency shift on the received reflected waves, are described in Yugoslav patent application P 1852/89, in US patents 4,790,323, 4,771,787, 4,706,681 and 4,697,595, as well as "Properties of ultrasound catheters" by B. Breyer and B. Ferek-Petric in the book Intracavitary Ultrasound, Kluwer Inc., Doordrecht, 1991, Editors N. Bom and N. Roelant.

In this way one can obtain, using an appropriate frequency filtration, data about the flow within the sensitive volume of the Doppler system, thus in the proximity of a catheter. The advantage of this method is its directness and the disadvantage is the relatively high power consumption and sophisticated electronics.

Another method for bulk flow estimation is the thermodilution measurement where one uses thermometers mounted on a catheter to measure the rate of cooling of the blood stream after injection of some liquid of a different temperature. The advantage of this method is its simplicity and the disadvantage the relatively poor accuracy and time averaging of the measurement. This method is known in the art for over twenty years and is the result of the state of electronics in that period.

The present invention is an attempt to obtain a new method of intraluminal blood flow velocity measurement with low power consumption as in thermodilution method, but having frequency handling capability comparable in practical terms (for the purpose) with the Doppler method. The method is based on the physical principle of the fluid energy continuance described with Bernoulli equations. Though the present method is mathematically equivalent to the measurement by means of Pitot method and Venturi tube, the devices disclosed here are technologically different.

The object of this application is the problem of measurement of the blood flow in the vicinity of the catheter implanted within the blood vessel or heart. The flow must be measured without additional disturbances with a frequency spectrum which covers the relevant frequencies found in the flow. The power consumption must be low and therefore the system must be passive i.e. it should not transmit any energy into the body. The system must be small enough to be built on a catheter of 2.7 mm diameter or larger, rugged enough to enable the implantation procedures with a standard venous introducer. System proportions should not impede the catheter flexibility and therefore the rigid section may not be longer than 1.5 cm. The system must be insensitive to the change of the atmospheric or body pressure. The system may not cause erythrocyte trauma, or thrombocyte reaction as well as cholesterol sedimentation.

The technological problem previously described has been solved using the device as indicated in claim 1 and in the subclaims.

The features of the present invention will be more readily understood by reference to the following description and accompanying drawings in which:
Fig. 1 represents a perspective view of a lead for cardiac electrostimulation (pacemaker lead) comprising two assemblies of transducers for blood velocity measurement.
Fig. 2 is a detailed partial perspective projection illustration of a device for axial flow velocity measurement comprising two sets of transducer assemblies mounted on a reinforcement tubelet which is incorporated within a pacemaker lead. One set of the transducer assemblies comprises a hydrofoil or aeroprofile form protrusion. The transducer sets consist of two or more segments, each segment having a shape of a fragment of the hollow cylinder.
Fig. 3 is a longitudinal cross section of the device of Fig. 2 in an autocompensation embodiment for the transducers connection with electric signals connected in counter-phase.
Fig. 4 is a longitudinal cross section of the measurement device of Fig. 2 with independent transducers in two sets. In this embodiment the signals from the static and that from the dynamic transducer set are independent and must be compared in an electronic system.
Fig. 5 is a transverse cross section showing the self compensating version of the transducer set as per Fig. 3. Segmentation and interconnection of the transducer set is illustrated. In the shown embodiment the interconnection of the internal electrodes is achieved using conductive glue onto the conductive reinforcement tubelet while the outer electrodes are connected with an elastic conductive foil.
Fig. 6 is a different embodiment of the measurement device of Fig. 2, wherein the elastic ring connection disclosed in Fig. 2 has been substituted with a wire or strip connection bridge.
Fig. 7 shows two modes of connection of the measurement device assemblies from previous Figs. 3 and 4 to electronic circuits for comparation and amplification of the signals. The electronic circuit is essentially a differential amplifier which amplifies the difference of the signals from the two piezoelectric transducer sets.

The detailed description of the devices illustrated within the figures are now described as follows:
The hydrodynamic system for blood flow measurement is based on the solution of the hydrodynamic problem using the Bernoulli equation. Two sets of piezoelectric transducers are mounted on an intraluminal device (i.e. catheter) close to each other or at a small distance between each other, e.g. 4 mm, if the axial length of the transducers is about 4 mm. One transducer set is mounted in such a way as not to disturb the flow and stream around the catheter. It is covered with a flexible insulating and waterproof membrane of a thickness less than 0.1 mm. This set is therefore exposed to the pressure of the surrounding liquid. It is called in "static transducer set" in this specification.

Another set of transducers is mounted in the same way, but has on its outer surface a protrusion made of plastic material. The protrusion has a cross sectional form of laminar flow hydrofoil or airfoil (e.g. NACA 4512, according to National Advisory Commitee on Aeronautics), which increases the flow velocity in the immediate vicinity inducing thereby a lift force as predicted by Bernoulli equation. This transducer set is called "dynamic transducer set" in this specification.

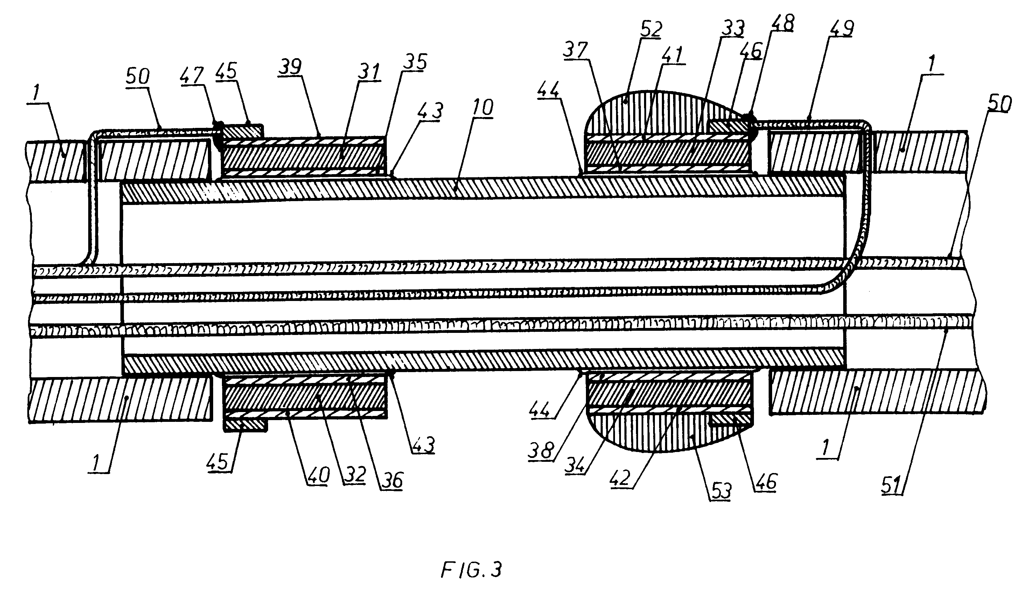

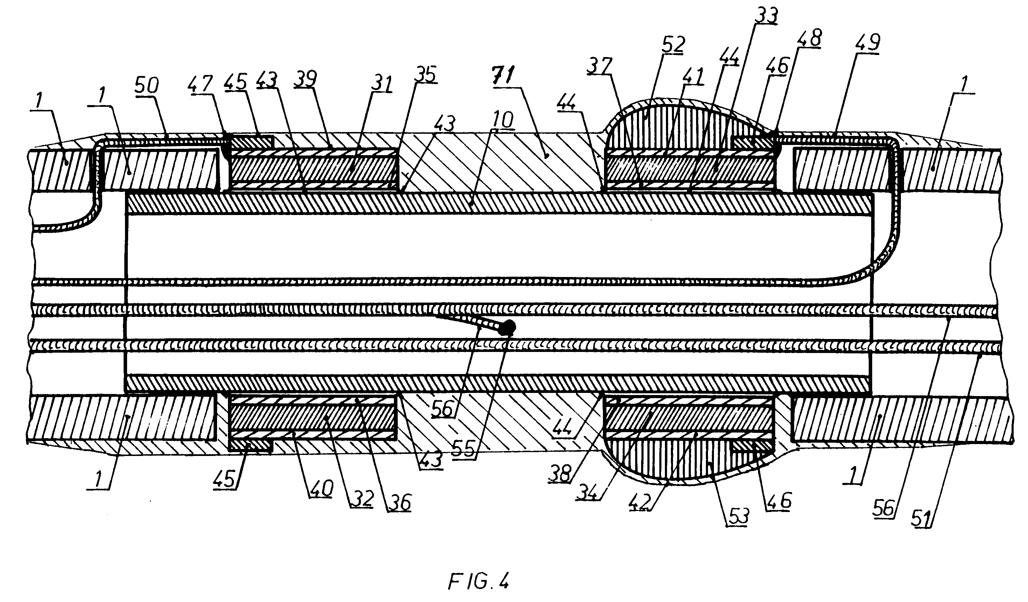

The lift force and the induced drag force are proportional to the square of the fluid velocity. The sensitivity of this dynamic transducer set to hydrostatic pressure is equal to that of the said static transducer set. In order to cancel the influence of the static pressure which is much larger than the hydrofoil lift, the transducer sets can be connected in opposite phases (senses) as shown on Fig. 3 or connected independently (Fig. 4) and taken to a differential amplifier.

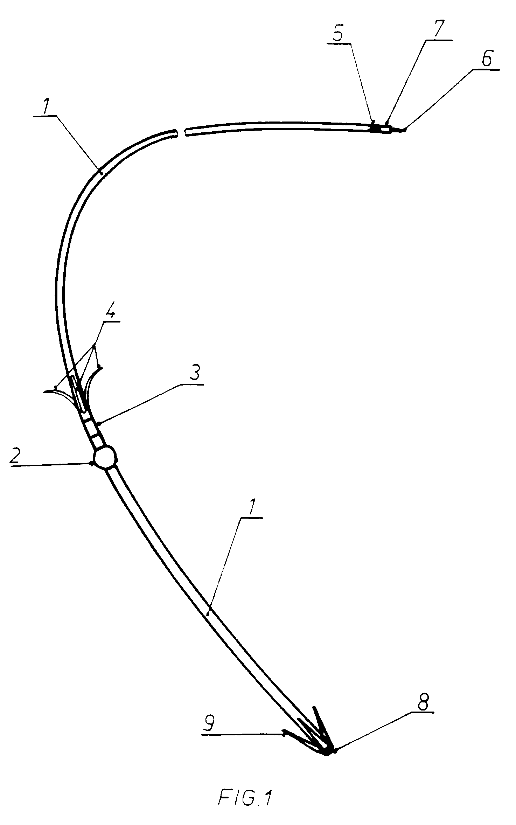

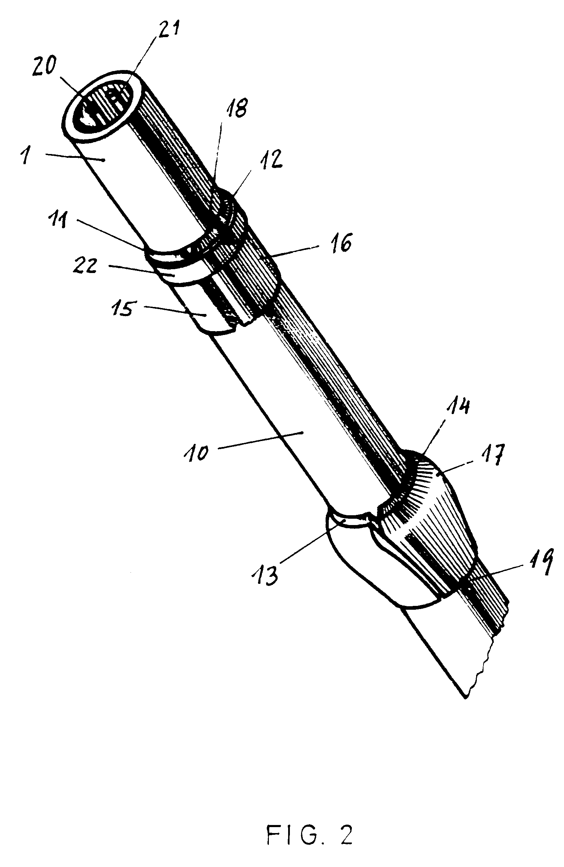

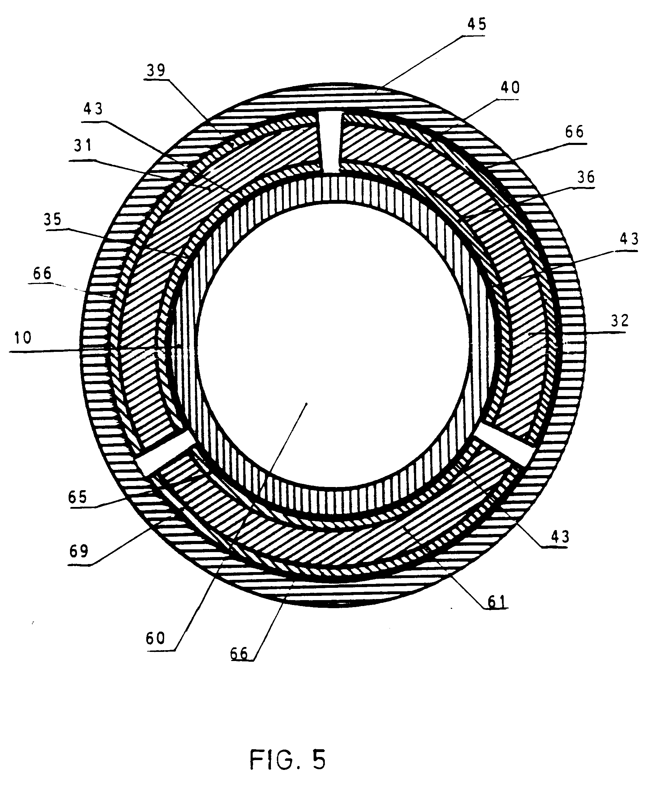

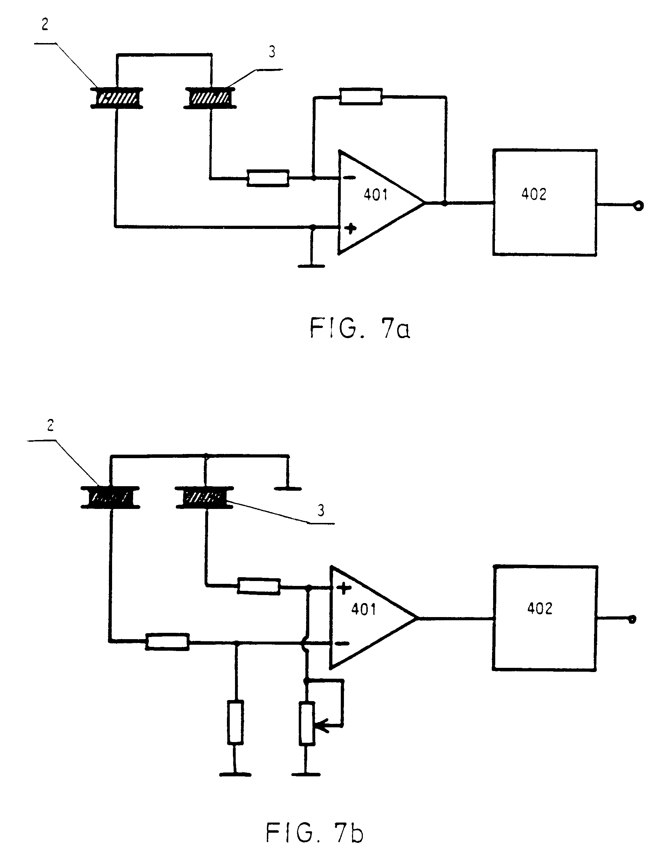

Mounting of the measurement system is illustrated in Fig. 1, some details of the transducer sets and their external appearance in Fig. 2, the longitudinal cross section of the compensational connection in Fig. 3, the longitudinal cross section of independent connection in Fig. 4, the transverse cross section in Fig. 5 and the connection to the electronic system in Fig. 7.

As shown in Fig. 1, two sets of piezoelectric transducers 2 and 3 are mounted onto a catheter 1. The part of the catheter where the transducers are mounted is reinforced with a reinforcing metal or plastic tube. A hydrofoil profile is mounted on one of the transducer sets 2, (the dynamic).

If the catheter is used for measuring the blood flow in a blood vessel, for instance in vena cava superior, a centralizing means 4 is provided in order to centralize the position of the catheter in the area of the transducer sets, because the transducer sets should be surrounded by the blood flow and should not touch the wall of the vessel. Such a centralizing means can be realized by some elastic fins fixed peripherally and adjacent to the transducer sets to the catheter so as to prevent major turbulences. If the catheter is used for measuring the blood flow within the heart, for instance the tricuspid flow, as in case of the cardiac pacing lead described in EP-A-474 957 that is part of the prior art under Article 54(3) EPC, then such a centralizing means 4 is not necessary.

The transducers are connected to electrical connectors 5, 6, 7 via built-in electrical conductors which can partly serve to carry the measurement and electrophysiologic signals as well. An electrode 8 is anchored to the endocardium with fins 9 if the catheter is designed as cardiac pacing lead.

Fig. 2 is a detailed perspective drawing of an embodiment of the measurement transducer assembly, wherein the protective layer covering the area which comprises the transducer sets, is removed. The two transducer sets are mounted onto a reinforcing tubelet 10 made of plastic or metal of a size fitting into the catheter 1. Tubelet 10 serves as a rigid support for the transducers in order to produce the reacting force for the forces to be detected. The transducer sets comprise piezoelectric segments 11/12 and 13/14 of a cylinder conductively glued or soldered onto the reinforcing tubelet 10. The device is axially symmetrical. All properties valid for one of the segments of any of the part transducers are valid for other segments. On the outer and the inner side of the said transducer segments there are thin (fired-on) electrodes; for the transducer segment 11 this is electrode 15 and for the other neighboring transducer segment 12 this is electrode 16. One set of the transducer segments 11, 12 has a cylindrical outside profile and is only covered with an insulating membrane (not shown). The other set of the transducer segments 13, 14 has a glued hydrofoil-like protrusion 17. Difference between flow induced signals is due to this hydrofoil element 17. Electrical connections are, in this embodiment, realized with conductors 18 and 19 which take the signal into the catheter body and lengthwise conductors 20 and 21. The electrical connection among the outside electrodes of the said transducer segments is, in this embodiment, realized using elastic conductive rings 22. The conductive ring provided in the transducer set supplied with the hydrofoil is not visible. The connection of the internal transducer electrodes goes via the conductive reinforcement tubelet 10.

In Fig. 3 is shown a lengthwise cross section of the measurement part of Fig. 2. This is the self-compensating measurement system embodiment. In this illustration the protective layer is removed, too. The body of the pacing lead holds the reinforcement device 10. Piezoelectric transducer segments 31, 32, 33, 34 which correspond to the transducer segments 11, 12, 13, 14 of Fig. 2 have inner fired-on sheath electrodes 35, 36, 37, 38 and outer fired-on electrodes 39, 40, 41, 42. In the actual embodiment there are three to four transducer segments per transducer set, so that in a lengthwise section one can not show all the segments, which is not necessary, knowing the axial symmetry of the device. The transducer segments are conductively glued or soldered by connections 43, 44 to the conductive reinforcement tubelet 10. The outer electrodes of the transducer segments are mutually electrically connected with elastic conductors (elastic ring 45 for transducer set containing transducer segments 31 and 32 and elastic ring 46 for transducer set containing transducer segments 33 and 34). The said elastic conductive rings are further connected by gluing or soldering through connections 47 and 48 to the lengthwise conductors 50 or 49, which take the resultant signal to the outer electronic circuits. Inside of the pacing lead there are other conductors 51 as well e.g. for the basic pacing purpose. The special feature of this invention is the hydrofoil profile or aeroprofile protrusion 52 and 53 (like NACA 4512 or Goettingen laminar profiles) which induces a difference in the signals, generated by the two transducer sets 31/32 and 33/34, which is proportional to the square of the axial flow velocity, the signal appearing between the conductors 49 and 50.

Fig. 4 shows the lengthwise cross section of a non self-compensating device. This embodiment differs from the embodiment in Fig. 3 in that it is differently electrically connected. The physical elements are indicated by numbers equal to those of Fig. 3.

Whereas in the illustration of Fig. 2, 3, 5 and 6 the protective layer is removed in case of Fig. 4 a protective layer 71 covering the area which comprises the transducer sets is illustrated. This protective layer consists of an electrically insulating material and serves two purposes, namely first to electrically insulate the outer electrodes 39, 40 of the transducer segments, the connecting ring 45 and conductors 49, 50 against the surrounding blood flow and secondly to provide a smooth profile of the catheter having only the hydrofoil profile 52/53 of the dynamic transducer set protruding therefrom. Because in this embodiment the two transducer sets are spaced apart from each other the axial gap between the transducer sets has to be filled up by the insulating material of the protective layer, e.g. in a moulding process or by an insulating ring (not shown) before covering the whole area with the protective layer for instance in a spraying process.

The difference compared with the device according to Fig. 3 consists of the conductor 56 soldered to or conductively glued by connections 55 to the reinforcement tubelet 10. This results in three conductors 49, 50, 56 which take the transducer signals to the outside electronics. The signals are in this case independent and can be combined in outside electronic circuits (refer to Fig. 7).

Fig. 5 is a transverse cross section through the said static transducer set. The lumen 60 of the pacing lead can accommodate all the needed lengthwise conductors (not shown). The piezoelectric transducer segments 31, 32, 61 are mechanically independently glued by connections 43 to the reinforcement tubelet 10. Their inner electrodes 35, 36, 65 are electrically mutually connected by means of the metallic reinforcement tubelet 10. Their external electrodes 39, 40, 69 are mutually electrically connected using the elastic conductive ring 45. The conductive ring can be realized as a metallized plastic foil, less than 10 »m thick. In order to obtain the illustration, conductive ring is shown in Fig. 5 in unreal thicker proportion. It is very important to note that the said hydrofoil projection glued onto the other dynamic transducer set is segmented in the same way as the shown transducer segments 31, 32, 61, i.e. has the same number of segments. Furthermore it is understood that the number of segments must be two or more, preferably three. If the transducer set would be designed as a closed cylindrical ring small radial forces could not be detected because of the rigidity of the ring.

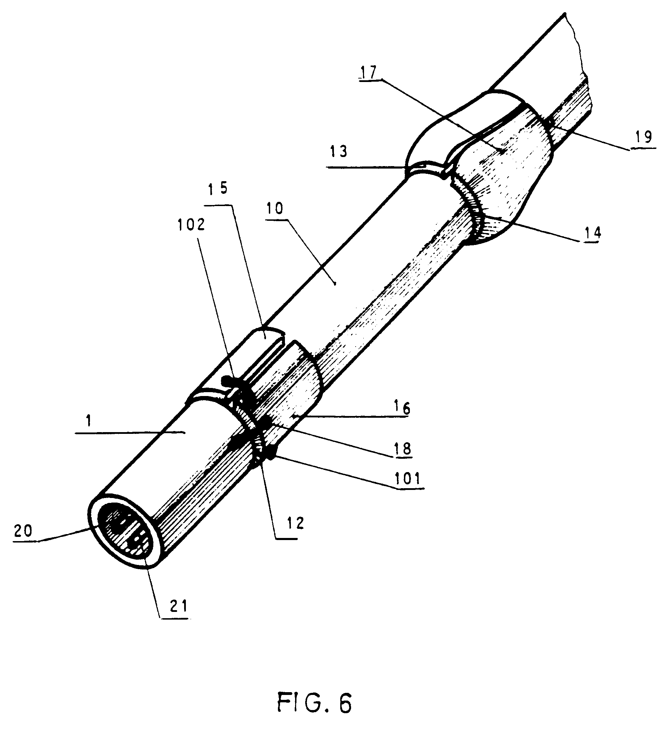

Another embodiment of the physical connection of the transducer system is disclosed on Fig. 6 wherein the conductive ring from previous figures has been substituted with interconnection bridges 101 and 102 made of knitted wire or strip. Because of the perspective view, Fig. 6 discloses only two of said bridges, though it is understood that all the outer piezoelectric transducer electrodes are connected by means of these bridges.

The electronic measurements system is basically disclosed in Fig. 7. Signals generated by the said transducer set 2 and 3 are led to the differential amplifier 401. Fig. 7a shows the basic electronic circuit for measurement of axial velocity by transducer sets as from Fig. 3, while Fig. 7b shows the basic electronic circuit for use of the signal from the device connected according to Fig. 4. In the connection of Fig. 7a the static signals from the two transducer sets cancel within the catheter, so only their difference is taken to the amplifier 401. In the embodiment of Fig. 6b the static component of signals from the both transducer sets are subtracted within the amplifier. Amplifier 401 is a stable operational amplifier as known in electronic technology. The output signal of the amplifier is taken into the circuits for signal processing 402 (filtering and ADC) in order to extract the required information for particular use.

### Example of a method for manufacturing and industrial application fo the invention

The basic task is construction of a catheter or other intraluminal device with built in static and hydrodynamic transducers. The basic materials are as follows: a catheter with built in electrical conductors, tubiform piezoceramic of PVDF transducers, plastic mass which can be formed (polystyrene, polyurethane, metylmetacrylate and their variations which satisfy the conditions). Electrical conductors in the catheter must be flexible and thin (copper or bronze insulated with PTFE) of a diameter in the order of magnitude of 0.1 mm. Conductors must in principle be built as multiple or spiralling conductors as conventional in the technology of pacing leads. Electrocatheters according to the previous requests can commercially be acquired from pacing lead manufacturers as "raw" catheters. One builds in a reinforcement at the position of intended measurement point, best in the form of aluminum, copper, brass or titanium alloy. Brass is satisfactory, in the form of a tube of an outer diameter of 2.7 mm and a wall thickness of 0.1 mm. Such a tubelet can commercially be acquired at the vehicle antenna producer. The building in is performed while the catheter is constructed by means of a thermoplastic deformation of the catheter. The most simple heating of a catheter is done using hot water. The tubelet is, after pulling on the catheter, covered with slowly setting (24 hours) two component conductive resin (e.g. manufactured by Doduco KG, Pforzheim), of a thickness in the order of magnitude of 0.05 mm. The piezoelectric transducers are glued in segmental form onto this sheath after all the contacts explained in the embodiment section have been made. These contacts can be obtained by putting electrical conductors which are connected to the internal side onto the half set resin (not less than 20 minutes after application) of the reinforcement tube. It is understood that the transducer segments have conductive electrodes on their internal side. If these electrodes are connected to the outer side of the segments, the described connections are formed by soldering in the way known in the transducer technology or by conductive gluing (e.g. with products of Doduco KG, Pforzheim or CIBA, Basel). Onto the outer surface-electrode of one of the transducer sets one glues on a hydrodynamic profile made of one of the previously said materials (silicone rubber, PMMA etc) obtained by moulding or machine made (lathe or grind). The form of the profile is taken from the coordinates tabulated in the tables for design of airfoils, e.g. "I.H. Abbot and A.E. VonDoenhoff: Theory of wing sections, Dover Publications". The manufacture is done with methods usual in the manufacture of transistors, i.e. with magnifying glass, laboratory stereomicroscope and micromanipulators used in classic semiconductor industry. The whole set, including both transducer sets is thereafter covered with an elastic electrically insulating sheath (made of latex or polyuretane) whereby a gap between the transducer set is filled up in order to obtain a smooth profile of the catheter having only the hydrofoil profile of the dynamic transducer set protruding therefrom. For small series it is sufficient to do the covering manually with a paintbrush or by spraying and subsequent heating at 40° in normal dry air heating chamber. The conductors are proximally being connected to appropriate connectors (e.g. banana plugs 2 mm in diameter, or pacemaker connector pins according to the standards), or are directly soldered onto the electronic amplifier. The complete lead is thereafter sterilized with radiation or chemically as customary in the manufacture of single use medical devices.

The device according to the invention can be used for blood flow measurement in a blood vessel, like an aorta, or in the heart. Blood flow velocity and changes thereof can be detected and the detected signals used for instance in order to control a cardiac pacing lead as it is described in EP-A-474 958 mentioned above. Flow measurement in an absolute manner can be carried out after a calibration of the device. Various waves as well as relative changes of amplitudes as well as accelerations can be detected.

## Claims

1. A hydrodynamic measuring device for blood flow measurement, comprising two sets (11/12, 13/14) of piezoelectric transducers mounted axially spaced apart from each other or axially adjacent to each other on a catheter (1) which is insertable into a blood vessel, where one of the said sets (13/14) is provided with a hydrofoil-like protrusion (17) on its external side so as to protrude from the catheter profile, while the second does not have such a protrusion, the two sets being connected to electrical conductors having connectors (21, 22) at the proximal end of the catheter.

2. Device according to claim 1, where the transducer sets (11/12, 13/14) are mounted on a reinforced section of said catheter (1).

3. Device according to claim 2, wherein said reinforced section is a tubelet (10), inserted into the catheter (1).

4. Device according to claim 3, wherein said tubelet (10) is made of rigid plastic or metal.

5. Device according to any of claims 1 to 4, wherein each of said transducer sets (11/12, 13/14) comprises at least two segments (11 and 12, 13 and 14).

6. Device according to claim 5, wherein each of said transducer sets (11/12 or 13/14) has the same number of segments (11 and 12, 13 and 14).

7. Device according to claim 5 or 6, wherein the segments (11 and 12, 13 and 14) of a transducer set (11/12 or 13/14) are arranged circumferentially with regard to the catheter (1).

8. Device according to any of claims 5 to 7, wherein all co-phase electrodes of the segments (11 and 12, 13 and 14) of a transducer set are electrically connected.

9. Device according to any of claim 1 to 8, wherein the co-phase electrodes of the two transducer sets (11/12, 13/14) are counter connected (Fig.3 and 7a).

10. Device according to any of claims 1 to 8, wherein the electrodes of the two transducer sets (11/12, 13/14) are connected via said conductors (49, 50, 56) to a differential or asymmetric electronic amplifier (Fig. 4 and 7b).

11. Device according to any of claims 1 to 10, wherein the catheter (1) is provided with centering means (4) in the neighbourhood of a transducer set.

12. Device according to any of claims 1 to 11, wherein said transducer set (13/14) which is provided with said hydrofoil-like protrusion is arranged in a downstream position with regard to the other transducer set (11/12) when inserted in a blood vessel or within the heart.

## Patentansprüche

1. Hydrodynamische Meßeinrichtung zur Blutströmungsmessung, mit zwei Sätzen (11/12, 13/14) piezoelektrischer Wandler, die axial im gegenseitigen Abstand voneinander oder axial benachbart zueinander an einem Katheter (1) angebracht sind, der in ein Blutgefäß einführbar ist, wobei einer dieser Sätze (13/14) an seiner Außenseite mit einem stromlinienförmigen Vorsprung (17) versehen ist, so daß er aus dem Katheterprofil vorsteht, während der zweite keinen solchen Vorsprung aufweist und daß die beiden Sätze mit elektrischen Leitern verbunden sind, die am naheliegenden Ende des Katheters Anschlüsse (21, 22) aufweisen.

2. Einrichtung nach Anspruch 1, bei der die Wandlersätze (11/12, 13/14) an einem verstärkten Abschnitt des Katheters (1) angebracht sind.

3. Einrichtung nach Anspruch 2, bei der der verstärkte Abschnitt ein in den Katheder (1) eingefügtes Röhrchen (10) ist.

4. Einrichtung nach Anspruch 3, bei der das Röhrchen (10) aus steifem Plastikmaterial oder Metall hergestellt ist.

5. Einrichtung nach einem der Ansprüche 1 bis 4, bei der jeder der Wandlersätze (11/12, 13/14) wenigstens zwei Segmente (11 und 12, 13 und 14) umfaßt.

6. Einrichtung nach Anspruch 5, bei der jeder der Wandlersätze (11/12 bzw. 13/14) die gleiche Anzahl an Segmenten aufweist (11 und 12, 13 und 14).

7. Einrichtung nach Anspruch 5 oder 6, bei der die Segmente (11 und 12, 13 und 14) eines Wandlersatzes (11/12 bzw. 13/14) bezuglich des Katheders (1) in Umfangsrichtung angeordnet sind.

8. Einrichtung nach einem der Ansprüche 5 bis 7, bei der alle gleichphasigen Elektroden der Segmente (11 und 12, 13 und 14) eines Wandlersatzes elektrisch miteinander verbunden sind.

9. Einrichtung nach einem der Ansprüche 1 bis 8, bei der die gleichphasigen Elektroden der beiden Wandlersätze (11/12, 13/14) einander entgegengeschaltet sind (Fig. 3 und 7a).

10. Einrichtung nach einem der Ansprüche 1 bis 8, bei der die Elektroden der beiden Wandlersätze (11/12, 13/14) über die Leiter (49, 50, 56) mit einem Differential-Verstärker oder einem asymmetrischen elektronischen Verstärker (Fig. 4 und 7b) verbunden sind.

11. Einrichtung nach einem der Ansprüche 1 bis 10, bei der der Katheder (1) benachbart zu einem Wandlersatz mit einer Zentriervorrichtung (4) versehen ist.

12. Einrichtung nach einem der Ansprüche 1 bis 11, bei der der mit dem stromlinienförmigen Vorsprung versehene Wandlersatz (13/14) stromabwärts bezüglich des anderen Wandlersatzes (11/12) angeordnet ist, wenn die Einrichtung in ein Blutgefäß oder innerhalb des Herzens eingeführt ist.

## Revendications

1. Dispositif de mesure hydrodynamique de l'écoulement de sang, comprenant deux jeux (11/12, 13/14) de transducteurs piézoélectriques montés axialement à distance l'un de l'autre ou axialement adjacents l'un à l'autre sur un cathéter (1) que l'on peut insérer dans un vaisseau sanguin, l'un de ces jeux (13/14) étant muni d'une protubérance (17) en forme d'aileron porteur sur son côté extérieur de manière à faire saillie du profil du cathéter, le second de ces jeux n'ayant pas une telle protubérance, les deux jeux étant reliés à des conducteurs électriques munis de connecteurs (21,22) à l'extrémité proximale du cathéter.

2. Dispositif selon la revendication 1, dans lequel les jeux (11/12, 13/14) de transducteurs sont montés sur une partie renforcée du cathéter (1).

3. Dispositif selon la revendication 2, dans lequel cette partie renforcée est un tubule (10), inséré dans le cathéter (1).

4. Dispositif selon la revendication 3, dans lequel ce tubule est en matière plastique rigide ou en métal.

5. Dispositif selon l'une quelconque des revendications 1 à 4, dans lequel chacun des jeux (11/12, 13/14) de transducteurs comprend au moins deux segments (11 et 12, 13 et 14).

6. Dispositif selon la revendication 5, dans lequel les jeux (11/12 ou 13/14) de transducteurs ont chacun le même nombre de segments (11 et 12, 13 et 14).

7. Dispositif selon la revendication 5 ou 6, dans lequel les segments (11 et 12, 13 et 14) d'un jeu (11/12 ou 13/14) de transducteur sont agencés circonférentiellement au cathéter (1).

8. Dispositif selon l'une quelconque des revendications 5 à 7, dans lequel toutes les électrodes en concordance de phase des segments (11 et 12, 13 et 14) d'un jeu de transducteurs sont reliées électriquement.

9. Dispositif selon l'une quelconque des revendications 1 à 8, dans lequel les électrodes en concordance de phase des deux jeux (11/12, 13/14) de transducteurs sont couplées de manière antiparallèle (figures 3 et 7a).

10. Dispositif selon l'une quelconque des revendications 1 à 8, dans lequel les électrodes des deux jeux (11/12, 13/14) de transducteurs sont reliées par l'intermédiaire des conducteurs (49,50,56) à un amplificateur électronique dissymétrique ou différentiel (figures 4 et 7b).

11. Dispositif selon l'une quelconque des revendications 1 à 10, dans lequel le cathéter (1) est muni de moyens (4) de centrage à proximité d'un jeu de transducteurs.

12. Dispositif selon l'une quelconque des revendications 1 à 11, dans lequel le jeu (13/14) de transducteurs qui est muni de la protubérance en forme d'aileron porteur est disposé en aval par rapport à l'autre jeu (11/12) de transducteurs lorsqu'ils sont insérés dans un vaisseau sanguin ou dans le coeur.
